# FASCICULE DE BREVET EUROPEEN

(11) **EP 1 406 627 B1**
(45) Date de publication et mention de la délivrance du brevet: **08.11.2006**
(21) Numéro de dépôt: 02770024.4
(22) Date de dépôt: 11.07.2002
(51) Int. Cl.: A61K 31/4545, A61P 25/36

(54) **DERIVES DE PYRIDIN-2-YL-METHYLAMINE POUR LE TRAITEMENT DE LA DEPENDANCE AUX OPIOIDES**
PYRIDIN-2-YL-METHYLAMIN-DERIVATE ZUR BEHANDLUNG VON OPIATSUCHT
PYRIDIN-2-YL-METHLYAMINE DERIVATIVES FOR TREATING OPIOID DEPENDENCE

(30) Priorité: 13.07.2001 FR 0109350
(43) Date de publication de la demande: 14.04.2004
(73) Titulaire: PIERRE FABRE MEDICAMENT, 92100 Boulogne (FR)
(72) Inventeur: COLPAERT, Francis, F-81700 Puylaurens (FR); BRUINS SLOT, Liesbeth, F-81100 Castres (FR); KOEK, Wouter, San Antonio, TX 78232 (US); TARAYRE, Jean-Pierre, 81100 Castres (FR); VACHER, Bernard, F-81100 Castres (FR)
(74) Mandataire: Warcoin, Jacques
(86) Numéro de dépôt international: PCT/FR2002/002449
(87) Numéro de publication internationale: WO 2003/006020

(56) Documents cités:
- EP-A- 0 356 997
- US-A- 6 020 345
- US-B1- 6 221 858
- GULATI A ET AL: "DOWN-REGULATION OF HYPOTHALAMIC 5 HT-1A RECEPTORS IN MORPHINE-ABSTINENT RATS" EUROPEAN JOURNAL OF PHARMACOLOGY, vol. 182, no. 2, 1990, pages 253-260, XP001064552 ISSN: 0014-2999
- BERTHOLD H ET AL: "MECHANISM OF THE ATTENUATION OF NALOXONE-INDUCED JUMPING BEHAVIOR IN MORPHINE DEPENDENT MICE BY 5 HT-1 RECEPTOR AGONISTS" BRITISH JOURNAL OF PHARMACOLOGY, vol. 95, no. SUPPL., 1988, page 869P XP001064547 ISSN: 0007-1188
- GONZALEZ J P ET AL: "BEHAVIORAL EFFECTS OF 5 HYDROXYTRYPTAMINE-1 AND 5 HYDROXYTRYPTAMINE-2 AGONISTS ON NALOXONE PRECIPITATED MORPHINE ABSTINENCE" BRITISH JOURNAL OF PHARMACOLOGY, vol. 86, no. SUPPL., 1985, page 444P XP001064551 ISSN: 0007-1188

## Description

La présente invention a pour objet une nouvelle utilisation de certains composés de la Pyridin-2-yl-méthylamine pour le traitement de la pharmacodépendance.

La pharmacodépendance à une substance psycho-active est un phénomène pathologique, classé parmi les troubles mentaux dans la classification internationale de l'Organisation Mondiale de la Santé. La liste des substances qui entraînent un syndrome de pharmacodépendance ou de dépendance est longue. Toutefois, le champ d'application de la présente invention se limite au traitement des pharmacodépendances aux opioïdes. Par opioïdes les inventeurs entendent l'ensemble des composés naturels, semi-synthétiques ou synthétiques dotés d'une activité morphinique.

Le principe du traitement de la pharmacodépendance est de permettre au sujet dépendant de devenir, puis de rester abstinent à la substance pour laquelle il est dépendant. Dans le cas particulier des dépendances aux opioïdes, les traitements pharmacologiques utilisés sont des traitements dit substitutifs. Leur principe consiste à remplacer la ou les substance(s) responsable(s) de la dépendance par un produit opioïde dont les effets spécifiques sont moindres et la durée d'action plus longue que ceux de la ou des substance(s) responsable(s) de la dépendance. Les médicaments disponibles dans l'indication de substitution sont, selon les pays, le chlorhydrate de méthadone, le levo-alpha-acétylméthadol (LAAM) et la buprénorphine. Le taux de réussite de ces traitements est, cependant, limité. De plus, comme les produits utilisés dans les traitements de substitution appartiennent eux-mêmes à la classe pharmacologique des opioïdes, ils présentent eux aussi un risque direct ou indirect pour la santé publique. Ainsi, les produits de substitution peuvent induire un syndrome de dépendance et/ou augmenter le risque d'abus et de dépendance à d'autres substances telles que, par exemple, la cocaïne. Conjointement aux traitements de substitution, certains agonistes des récepteurs α2, tels que la clonidine et la lofexidine, sont aussi, parfois, utilisés en traitement de soutient et de prévention des rechutes après cure de sevrage aux opioïdes.

Les traitements actuels de la pharmacodépendance aux opioïdes ne sont donc pas entièrement satisfaisant. A ce titre, la découverte de nouveaux traitements mettant en oeuvre des molécules dotées d'un mécanisme d'action différent de celui des produits existants, est fortement souhaitable.

Les mécanismes qui sous-tendent le syndrome de dépendance sont complexes et, de ce fait, difficiles à cerner. En effet, différentes classes de récepteurs et de neurotransmetteurs sont impliqués. Il semble néanmoins établit que la dopamine joue un rôle central. Il ressort également que la noradrénaline et la sérotonine qui agissent, entre autres, comme modulateurs et/ou régulateurs de l'activité des neurones dopaminergiques dans les zones cérébrales impliquées dans les phénomènes de dépendance, constituent des cibles privilégiés d'interventions thérapeutiques.

En fait, l'existence d'un lien entre certains sous-types du site de liaison de la sérotonine, dont entre autres le sous-type 5-HT_{1A}, et la dépendance à la morphine a été discuté dans la littérature scientifique dès la fin des années 80. Consécutivement, le champ d'application potentiel des composés ayant une affinité pour le récepteur 5-HT_{1A} s'est vue, plus ou moins systématiquement, étendu au traitement du syndrome de sevrage et/ou de l'abus et/ou des pharmacodépendances à des substances psycho-actives, y compris l'éthanol et la nicotine.

Ainsi, la société Bristol-Myers Co. revendique dans la demande européenne EP 356997 l'utilisation d'azapirones, tel que par exemple la buspirone, dans le traitement de l'abus à des substances psycho-actives.

La société American Home Products Corporation décrit dans les demandes internationales WO 0035892, WO 0035874, WO 0035878 des dérivés de pipérazine-éthylamides, d'arylpiperidines et de 1,4-pipérazines, respectivement, comme agonistes et antagonistes du récepteur 5-HT_{1A} utiles dans le traitement de l'accoutumance aux drogues. Dans la demande internationale WO 9938864, la même compagnie décrit des dérivés d'oxazoles comme agonistes du récepteur 5-HT_{1A} utiles dans le traitement du syndrome de sevrage et de l'accoutumance aux drogues. Dans les demandes WO 9808817 et WO 9717343 des dérivés de 4-aminoethoxyindoles et de benzodioxanemethylamines, respectivement, sont revendiqués comme ligands du récepteur 5-HT_{1A} utiles dans le traitement de l'abus et de la dépendance. Dans la demande internationale WO 0035875 des dérivés d'arylpiperidines sont revendiqués comme antagonistes du récepteur 5-HT_{1A} utiles dans le traitement de l'accoutumance aux drogues.

La société Knoll revendique dans les demandes internationales WO 9723485, WO 9702269, 9703071 des dérivés d'hétéroarylcarboxamides, de thiazoles, d'hétérocyclylcarboxamides, respectivement, comme ligands non sélectifs du récepteur 5-HT_{1A} utiles dans le traitement de l'abus et de l'accoutumance aux substances psycho-actives.

La société Pharmacia & Upjohn S.P.A. rapporte dans la demande internationale WO 9730050 des dérivés d'hétérocyclyl-ergolines comme ligands sélectifs du récepteur 5-HT_{1A} utiles dans le traitement du sevrage et de l'accoutumance aux drogues; également, dans la demande WO 9741858 des dérivés de pipérazines sont décrits comme ligands non sélectifs du récepteur 5-HT_{1A} utiles dans le traitement de l'abus de substances psycho-actives.

La société Pfizer Products Inc. décrit dans les demandes EP 982030 et EP 928792 des dérivés de 2,7-substitués-octahydro-pyrrolo-1,2-pyrazines et de Bicyclo(3.1.0)hexanes, respectivement, comme ligands du récepteur 5-HT_{1A} utiles dans le traitement de la dépendance.

La société R.P. Scherer Limited revendique dans la demande internationale WO 9842344 l'utilisation d'une composition pharmaceutique comprenant un agoniste 5-HT_{1A} tel que, par exemple, la buspirone, dans le traitement de l'abus et de l'accoutumance à certaines substances.

La société Synthélabo décrit dans la demande WO 9706155 des dérivés de naphtalen-1-yl-piperazines comme ligands du récepteur 5-HT_{1A} utiles dans le traitement des troubles du au sevrage ou à l'abus de stupéfiants.

La société Yamanouchi Pharm Co Ltd rapporte dans la demande internationale WO 9429293 des dérivés de fluorochromanes comme ligands du récepteur 5-HT_{1A} utiles dans le traitement de la dépendance aux drogues.

La société Eli Lilly and company décrit des hétéro-oxy-alkanamines dans la demande US 5741789 comme étant des agonistes ou des agonistes partiels du récepteur 5-HT_{1A} utiles dans le traitement de l'abus de substances. Dans la demande internationale WO 0000196, la même société rapporte des pyrrolidines et des pyrrolines comme étant des antagonistes non sélectifs du récepteur 5-HT_{1A} utiles dans le traitement de l'abus de substances.

Cependant, dans aucune des demandes de brevet citées ci-dessus, l'utilité des ligands 5-HT_{1A} dans le traitement des pharmacodépendances à des substances psycho-actives autres que l'éthanol (WO 9741858) n'est supportée par des données pharmacologiques. De plus, les agonistes 5-HT_{1A} testés cliniquement dans le traitement de la dépendance à certains stupéfiants tels que, par exemple, la cocaïne, ont donné des résultats négatifs. L'impact des composés ayant une activité pharmacologique du type agoniste 5-HT_{1A} sur le traitement de la pharmacodépendance aux opioïdes, est donc, jusqu'à présent, inexistant. Selon l'état actuel de la technique, l'utilité potentielle de nouveaux agonistes 5-HT_{1A} dans le traitement de la pharmacodépendance aux opioïdes est difficilement prévisible.

Les inventeurs ont découvert, de manière surprenante, que les composés revendiqués par la demanderesse dans le brevet WO 98/22459 et représentés par la formule générale (I) : 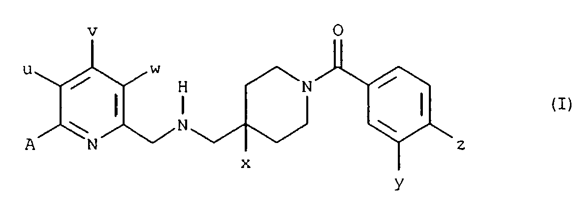 dans laquelle:
- **u**: représente un atome d'hydrogène ou un radical méthyl avec la réserve que lorsque **u** est un radical méthyl alors **v** et **w** représentent un atome d'hydrogène;
- **v**: représente un atome d'hydrogène, un atome de chlore ou un radical méthyl avec la réserve que lorsque **v** est un radical méthyl alors **u** et **w** représentent un atome d'hydrogène;
- w: représente un atome d'hydrogène, un atome de fluore ou un radical méthyl avec la réserve que lorsque w est un radical méthyl alors **u** et **v** représentent un atome d'hydrogène;
- **x**: représente un atome d'hydrogène ou un atome de fluore;
- **y**: représente un atome de chlore ou un radical méthyl;
- **z**: représente un atome d'hydrogène ou un atome de fluore ou un atome de chlore ou un radical méthyl;
- **A**: représente:
- un atome d'hydrogène ou un atome de fluore ou un atome de chlore;
- un radical alkyl en C₁-C₅, i.e. un reste d'hydrocarbure aliphatique saturé à chaîne droite ou ramifiée, contenant 1 à 5 atomes de carbone tel que méthyl, éthyl, propyl, butyl, pentyl, isopropyl, 1-méthyl-éthyl, 1-méthyl-propyl, 1-méthyl-butyl, 2-méthyl-propyl, 2-méthyl-butyl ou 3-méthyl-butyl, 1-éthyl-propyl, 2-éthyl-propyl;
- un radical fluoroalkyl tel que monofluorométhyl (-CH₂F) ou difluorométhyl (-CHF₂) ou trifluorométhyl (-CF₃) ou 1-fluoro-1-éthyl (-CHFCH₃) ou 1,1-difluro-1-éthyl (-CF₂CH₃);
- un radical cyclopropyl ou cyclobutyl ou cyclopentyl;
- un groupe hétérocyclique aromatique à 5 chaînons, substitué ou non, contenant 1, 2, 3 ou 4 hétéroatomes choisis parmis l'azote, l'oxygène et le soufre sans toutefois que plus d'un atome d'oxygène et / ou de soufre ne soit présent dans l'hétérocycle A.
- un groupe alkoxy (R₁O-) ou alkylthio (R₁S-) dans lequel le radical R₁ représente:
   · un radical alkyl en C₁-C₅ tel que défini précédemment,
   · un radical monofluorométhyl ou trifluorométhyl,
   · un radical cyclopropyl ou cyclobutyl ou cyclopentyl;
- un groupe amino de type II 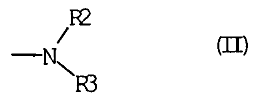
dans lequel R₂ et R₃, identiques ou différents représentent l'hydrogène, ou un radical alkyl en C₁-C₅ tel que défini précédemment ou un groupe cyclopropyl ou un groupe trifluorométhyl;
- un groupe amino cyclique saturé de type III 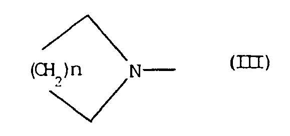
dans lequel n peut prendre les valeurs entières 1 ou 2;
- un groupe alkoxycarbonyl,de préférence un groupe méthoxycarbonyl (CH₃OCO-) ou un groupe éthoxycarbonyl (CH₃CH₂OCO-); ainsi que ses sels d'addition avec des acides minéraux ou organiques pharmaceutiquement acceptables sont, de manière inattendu, potentiellement efficaces dans le traitement de la pharmacodépendance aux opioïdes.

Les composés de formule **(I)** sont des agonistes sélectifs des récepteurs 5-HT_{1A} connus et leur utilisation dans d'autres domaines de la médecine sont décrites dans la demande internationale WO 98/22459. Dans la présente invention le composé de formule **(I)** préféré est le (3-Chloro-4-fluorophényl)-(4-fluoro-4-{[(5-méthyl-pyridin-2-ylméthyl)-amino]-méthyl}-pipéridin-1-yl)-méthanone fumarate, soit : **A** = H, **u** = CH₃, **v** et **w** =H, χ= F, **y** = CI et **z** = **F** dans la formule générale **(I).** Le composé en question est nommé composé F ci-après.

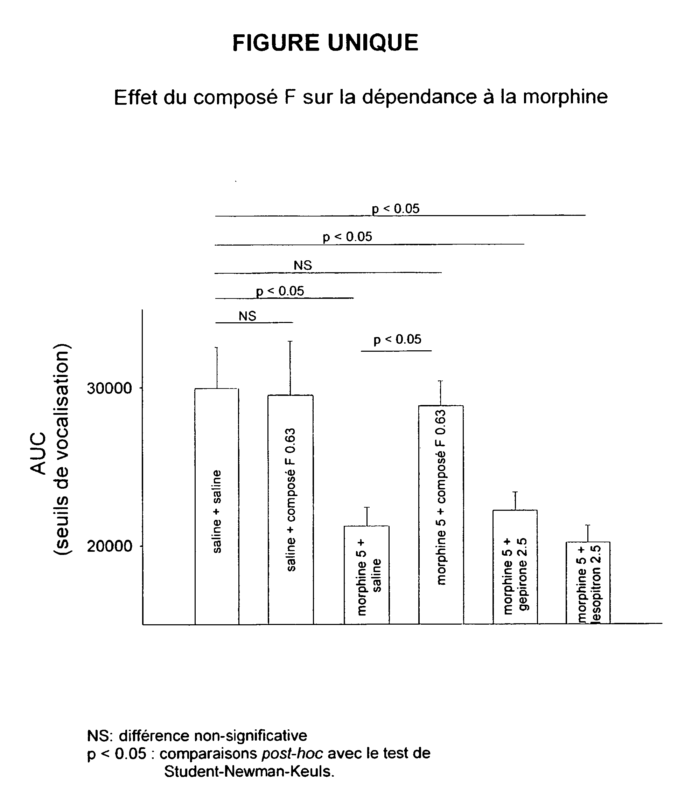

La figure unique montre l'effet du composé F sur la dépendance à la morphine.

La validité des modèles animaux pour l'étude des mécanismes neurobiologiques et comportementaux impliqués dans la pharmacodépendance s'appuie maintenant sur plus de vingt ans de recherche. Plusieurs aspects de la conduite des individus dépendants peuvent ainsi être reproduits chez l'animal de laboratoire. Par exemple, chez le rat rendu dépendant par administration chronique de morphine, l'interruption brutale de morphine ou l'administration d'un antagoniste des récepteurs opiacés induit un état dit hyperalgésique qui peut être facilement objectivé au moyen de stimuli nociceptifs. Ainsi, cet état d'hyperalgésie induit, constitue un marqueur sensible, robuste et largement reconnu de la dépendance aux opiacés. En conséquence, les inventeurs ont choisi d'utiliser un tel marqueur pour évaluer la capacité des composés de l'invention à s'opposer à l'apparition d'un syndrome de dépendance à la morphine.

Le composé F a été comparé à la gépirone et au lésopitron choisis comme agonistes 5-HT_{1A} de références. La gépirone [83928-76-1], qui est un proche analogue structural de la buspirone [33386-08-2] et de la tandospirone [112457-95-1], est un agoniste 5-HT_{1A} en essais cliniques de phase III. Le lesopitron [132449-46-8] est le seul agoniste 5-HT_{1A} en développement actif dans l'indication de pharmacodépendance (Pharmaprojects Mars 2001).

Les exemples suivants servent à illustrer l'activité pharmacologique du composé F et, donc, son utilité potentielle dans l'indication thérapeutique revendiquée dans l'invention.

### Exemple 1 : Mesure de l'affinité du composé F et des produits de référence pour le récepteur 5-HT_{1A}

L'étude de la liaison au récepteur 5HT_{1A} a été réalisée selon une méthode standard (Naunyn-Schmiedeberg's Arch. Pharmaco. 1991, 343, 106). Les constantes d'inhibition (Ki) des produits de l'invention sont estimées à partir des expérimentations de déplacement en utilisant le programme de régression non-linéaire RADLIG version 4 de EBDA (Equilibrium Binbing Data Analysis) (Biosoft, Cambridge, UK, Mc Pherson, 1985). Les constantes de dissociation des ligands radioactifs utilisées dans les calculs sont de 0.31 nmole pour ³H-8-OH-DPAT. Les valeurs de pKi (-logKi) sont données sous forme de moyenne ± SEM d'au moins 3 expérimentations.

Les résultats obtenus sont rapportés dans le tableau, ci-dessous.

| Composé | pKi site 5-HT_{1A}^{a)} |
|---|---|
| composé F | 9.07 |
| gépirone | 7.17 |
| lesopitron | 7.26 |

| | |
|---|---|
| ^{a)} Tissu : cortex cérébral de rat. | |

### Exemple 2 : Effets du composé F et des produits de référence sur la dépendance à la morphine

Les produits sont administrés au moyen de mini-pompes osmotiques (modèle 2ML2 ; débit 5 µL / h ; Alza Corporation, Palo Alto, USA) implantées en sous-cutanée le premier jour de l'expérimentation et enlevées deux semaines plus tard. La pompe est mise en place à travers une incision transversale effectuée dans la peau de la face dorsale du rat, l'orifice de libération étant dirigé vers la tête.

Le chlorhydrate de morphine est administrée à 5 mg/rat/jour (41.7 mg/ml) en solution dans de l'eau distillée. Le composé F est administrée à 0.63 mg/rat/jour (5.25 mg/ml) en solution dans de l'eau distillée. La gépirone et le lesopitron sont administrés à 2.5 mg/rat/jour (20.8 mg/ml) en solution dans de l'eau distillée. Les pompes implantées chez les animaux témoins libèrent 0.12 ml de 0.9% NaCl (saline)/rat/jour ; les doses se réfèrent au poids du composé non salifié.

L'étude consiste en deux phases : une phase de traitement chronique qui dure 2 semaines, et une phase de sevrage qui dure 1 semaine.

Au premier jour de l'expérience les rats sont implantés avec deux mini-pompes osmotiques. Six groupes expérimentaux reçoivent un des traitements suivants : (a) pompe 1 = saline et pompe 2 = saline (n = 11) ; (b) pompe 1 = saline et pompe 2 = composé F à 0.63 mg/rat/jour (n = 11) ; (c) pompe 1 = morphine à 5 mg/rat/jour et pompe 2 = saline (n = 13) ; (d) pompe 1 = morphine à 5 mg/rat/jour et pompe 2 = composé F à 0.63 mg/rat/jour (n = 13) ; (e) pompe 1 = morphine à 5 mg/rat/jour et pompe 2 = gépirone à 2.5 mg/rat/jour (n = 13) ; (f) pompe 1 = morphine à 5 mg/rat/jour et pompe 2 = lesopitron à 2.5 mg/rat/jour (n = 13).

Après deux semaines de traitement chronique commence la phase de sevrage. Après avoir pris des mesures basales au jour 14, tous les animaux reçoivent une injection sous-cutanée de naloxone à 0.63 mg/kg. Ensuite, 30 minutes, 1 h, 2h, 4h et 8h après l'injection, une autre série de mesures est prise. Les pompes sont explantées après la dernière mesure à 8h après l'injection de naloxone. Une série de mesures est également prise quotidiennement 24h à 4 jours après l'injection de naloxone. Pour cette phase de sevrage, les seuils nociceptifs sont mesurés en utilisant la méthode de Randall et Selitto (Arch. Int. Pharmacodyn. 1957, CXI, 409) dans laquelle une pression croissante est appliquée sur la patte arrière jusqu'à atteindre le seuil de vocalisation. Les résultats sont exprimés en grammes et une limite de 750 g est imposée.

Pour l'évaluation des seuils nociceptifs, les aires sous les courbes (AUCs) sont déterminées et traitées par une analyse de variance (ANOVA). Les comparaisons *post-hoc* (i.e., qui suivent l'analyse de variance lorsque celle-ci est significative) sont réalisées avec le test de Student-Newman-Keuls (SNK) pour une comparaison des groupes entre eux. Un effet statistiquement significatif est défini à p < 0.05. Les aires sous la courbe (AUCs) obtenues entre 30 minutes et 4 jours après l'induction du sevrage par la naloxone sont représentées dans le graphe donné en annexe.

### Résultats

Le résultat des tests de liaison in vitro (cf. tableau ci-dessus) montre que le composé F possède une forte affinité pour le récepteur du sous type 5-HT_{1A} L'affinité du composé F vis à vis de ce site est, de plus, très supérieure à celles des produits de référence : gépirone et lesopitron.

In vivo, les valeurs des AUCs (cf. la figure) significativement plus faibles chez les animaux ayant reçu de la morphine que chez ceux ayant reçu du sérum physiologique montrent l'apparition attendue du syndrome de sevrage à la morphine [ANOVA, effet traitement, F(5,68) = 5.7; p < 0.001]. La diminution significative (SNK; p < 0.05) des seuils nociceptifs des animaux ayant reçu de la morphine montre que ce syndrome peut être clairement objectivé par la mesure de l'amplitude de l'hyperalgésie qui se développe lors de la phase de sevrage. Les animaux co-traités par la morphine et le composé F, contrairement à ceux traités par la morphine seule et à ceux traités simultanément par la morphine et la gépirone (SNK; p < 0.05) ou le lesopitron (SNK; p < 0.05), présentent des valeurs d'AUCs statistiquement comparables (SNK; p > 0.05) à celles obtenues chez les animaux témoins. Le composé F n'induit par d'effet par lui-même, comme le montre les seuils comparables (p > 0.05) des animaux témoins et de ceux traités avec le composé F seul. Il s'ensuit que seuls les animaux co-traités par la morphine et le composé F ne développent pas d'hyperalgésie et ne présentent donc pas de syndrome de sevrage à la morphine. Les agonistes 5-HT_{1A} de référence (i.e., gépirone et lesopitron) n'ont pas d'effet détectable sur l'hyperalgésie et donc sur le syndrome de sevrage à la morphine. De ce fait, contrairement au composé de référence, le composé F est potentiellement capable de s'opposer à la dépendance à la morphine.

Il ressort de cette étude que les composés de formule (I) ainsi que ses sels d'addition avec des acides minéraux ou les acides organiques pharmaceutiquement acceptables sont, contrairement aux agonistes 5-HT_{1A} appartenant à d'autres classes chimiques, potentiellement utiles dans le traitement de la pharmacodépendance aux opioïdes.

Des compositions pharmaceutiques peuvent être preparées, contenant à titre de principe actif au moins un des dérivés de formule générale (I) ou un de ses sels ou hydrates de ses sels en combinaison avec un ou plusieurs excipients ou véhicules pharmaceutiquement acceptables.

Lesdites compositions pharmaceutiques peuvent être, à titre d'exemple, des compositions administrables par voie orale, nasale, sublinguale, rectale, ou parentérale. A titre d'exemple de compositions administrables par voie orale on peut citer les comprimés, les gélules, les granules, les poudres et les solutions ou suspensions orales.

Les formulations appropriées pour la forme d'administration choisie sont connues et décrites, par exemple dans : Remington, The Science and Practice of Pharmacy, 19^{ième} édition, 1995, Mack Publishing Company.

La dose efficace d'un composé de l'invention varie en fonction de nombreux paramètres tels que, par exemple, la voie d'administration choisie, le poids, l'âge, le sexe, la ou les substance(s) responsable(s) de la pathologie, la sensibilité de l'individu à traiter. En conséquence, la posologie optimale devra être déterminée individuellement, en fonction des paramètres pertinents, par un spécialiste du corps médical. Bien que les doses efficaces d'un composé de l'invention puissent varier dans de larges proportions, les doses journalières pourraient s'échelonner entre 0.01 mg et 100 mg par Kg de poids corporel de l'individu à traiter. Une dose journalière d'un composé de l'invention comprise entre 0.10 mg et 100 mg par Kg de poids corporel de l'individu à traiter étant, toutefois, préférée.

## Revendications

1. Utilisation d'un composé de formule (I) 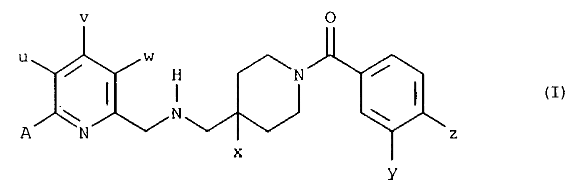 et de ses sels thérapeutiquement acceptables
dans laquelle:
**u** représente un atome d'hydrogène ou un radical méthyl avec la réserve que lorsque **u** est un radical méthyl alors **v** et **w** représentent un atome d'hydrogène;
**v** représente un atome d'hydrogène, un atome de chlore ou un radical méthyl avec la réserve que lorsque **v** est un radical méthyl alors **u** et **w** représentent un atome d'hydrogène;
**w** représente un atome d'hydrogène, un atome de fluore ou un radical méthyl avec la réserve que lorsque **w** est un radical méthyl alors **u** et **v** représentent un atome d'hydrogène;
**x** représente un atome d'hydrogène ou un atome de fluore;
**y** représente un atome de chlore ou un radical méthyl;
**z** représente un atome d'hydrogène ou un atome de fluore ou un atome de chlore ou un radical méthyl;
**A** représente:
- un atome d'hydrogène ou un atome de fluore ou un atome de chlore;
- un radical alkyl en C₁-C₅, i.e. un reste d'hydrocarbure aliphatique saturé à chaîne droite ou ramifiée, contenant 1 à 5 atomes de carbone tel que méthyl, éthyl, propyl, butyl, pentyl, isopropyl, 1-méthyl-éthyl, 1-méthyl-propyl, 1-méthyl-butyl, 2-méthyl-propyl, 2-méthyl-butyl ou 3-méthyl-butyl, 1-éthyl-propyl, 2-éthyl-propyl;
- un radical fluoroalkyl tel que monofluorométhyl (-CH₂F) ou difluorométhyl (-CHF₂) ou trifluorométhyl (-CF₃) ou 1-fluoro-1-éthyl (-CHFCH₃) ou 1,1-difluro-1-éthyl (-CF₂CH₃);
- un radical cyclopropyl ou cyclobutyl ou cyclopentyl;
- un groupe hétérocyclique aromatique à 5 chaînons, substitué ou non, contenant 1, 2, 3 ou 4 hétéroatomes choisis parmis l'azote, l'oxygène et le soufre sans toutefois que plus d'un atome d'oxygène et / ou de soufre ne soit présent dans l'hétérocycle **A**.
- un groupe alkoxy (R₁O-) ou alkylthio (R₁S-) dans lequel le radical R₁ représente:
· un radical alkyl en C₁-C₅ tel que défini précédemment,
· un radical monofluorométhyl ou trifluorométhyl,
· un radical cyclopropyl ou cyclobutyl ou cyclopentyl;
- un groupe amino de type II 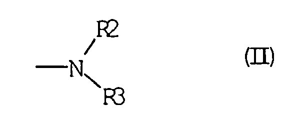
dans lequel R₂ et R₃, identiques ou différents représentent l'hydrogène, ou un radical alkyl en C₁-C₅ tel que défini précédemment ou un groupe cyclopropyl ou un groupe trifluorométhyl;
- un groupe amino cyclique saturé de type III 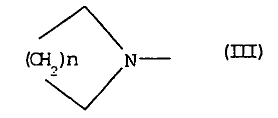
dans lequel n peut prendre les valeurs entières 1 ou 2;
- un groupe alkoxycarbonyl, de préférence un groupe méthoxycarbonyl (CH₃OCO-) ou un groupe éthoxycarbonyl (CH₃CH₂OCO) ; pour la préparation d'un médicament destiné au traitement de la pharmacodépendance aux opioïdes.

2. Utilisation d'un composé de formule (I) selon la revendication 1, **caractérisée en ce que** A représente un atome d'hydrogène, u représente un méthyl, v et w représentent un atome d'hydrogène, x représente un fluore, y représente un chlore et z représente un fluor.

## Claims

1. Utilisation of a compound of formula (I) 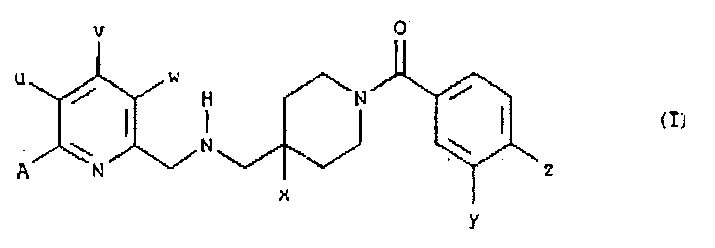 and its therapeutically acceptable salts in which:
u represents a hydrogen atom or a methyl radical with the reservation that when u is a methyl radical then v and w represent a hydrogen atom;
v represents a hydrogen atom, a chlorine atom or a methyl radical with the reservation that when v is a methyl radical then u and w represent a hydrogen atom;
w represents a hydrogen atom, a fluorine atom or a methyl radical with the reservation that when w is a methyl radical then u and v represent a hydrogen atom;
x represents a hydrogen atom or a fluorine atom;
y represents a chlorine atom or a methyl radical;
z represents a hydrogen atom or a fluorine atom or a chlorine atom or a methyl radical;
A represents:
- a hydrogen atom or a fluorine atom or a chlorine atom;
- an alkyl radical in C₁-C₅, i.e. a saturated aliphatic hydrocarbon remainder with straight or branched chain, containing from 1 to 5 atom of carbon such as methyl, ethyl, propyl, butyl, pentyl, isopropyl, 1-methyl-ethyl, 1-methyl-propyl, 1-methyl-butyl, 2-methyl-propyl, 2-methyl-butyl or 3-methyl-butyl, 1-ethyl-propyl, 2-ethyl-propyl;
- a fluoroalkyl radical such as monofluoromethyl (-CH₂F) or difluoromethyl (-CHF₂) or trifluoromethyl (-CF₃) or 1-fluoro-1-ethyl (-CHFCH₃) or 1,1-difluoro-1-ethyl (-CF₂CH₃) ;
- a cyclopropyl or cyclobutyl or cyclopentyl radical;
- an aromatic heterocyclic group with 5 links, substituted or not, containing 1, 2, 3 or 4 heteroatoms chosen from amongst nitrogen, oxygen and sulphur but nonetheless without more than one oxygen and/or sulphur atom being present in the heterocycle A;
- an alkoxy (R₁O-) or alkythio (R₁S-) group in which the R₁ radical represents:
- an alkyl radical in C₁-C₅ such as defined above;
- a monofluoromethyl or trifluoromethyl radical;
- a cyclopropyl or cyclobutyl or cyclopentyl radical;
- an amino group of type II 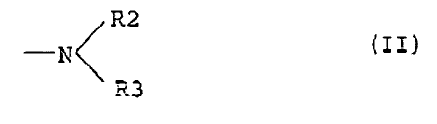
in which R₂ and R₃, identical or different, represent hydrogen or an alkyl radical in C₁-C₅ such as defined above or a cyclopropyl group or a trifluoromethyl group;
- a saturated cyclic amino group of type III 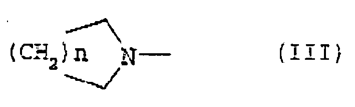
in which n can take the whole numbers 1 or 2;
- an alkoxycarbonyl group, preferably a methoxycarbonyl (CH₃OCO-) group or an ethoxycarbonyl (CH₃CH₂OCO) group; for preparing a medicament intended for the treatment of opioid drug dependence.

2. Utilisation of a compound of formula (I) according to claim 1, **characterised in that** A represents a hydrogen atom, u represents a methyl, v and w represent a hydrogen atom, x represents a fluorine, y represents a chlorine and z represents a fluorine.

## Patentansprüche

1. Verwendung einer Verbindung der Formel (I) 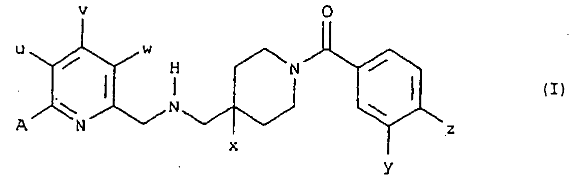 und ihrer therapeutisch annehmbaren Salze,
in der:
**u** ein Wasserstoffatom oder einen Methylrest darstellt, mit der Maßgabe, dass, wenn **u** ein Methylrest ist, dann **v** und **w** ein Wasserstoffatom darstellen;
**v** ein Wasserstoffatom, ein Chloratom oder einen Methylrest darstellt, mit der Maßgabe, dass, wenn **v** ein Methylrest ist, dann **u** und **w** ein Wasserstoffatom darstellen;
**w** ein Wasserstoffatom, ein Fluoratom oder einen Methylrest darstellt, mit der Maßgabe, dass, wenn **w** ein Methylrest ist, dann **u** und **v** ein Wasserstoffatom darstellen;
**x** ein Wasserstoffatom oder ein Fluoratom darstellt;
**y** ein Chloratom oder einen Methylrest darstellt;
**z** ein Wasserstoffatom oder ein Fluoratom oder ein Chloratom oder einen Methylrest darstellt;
**A** darstellt:
- ein Wasserstoffatom oder ein Fluoratom oder ein Chloratom;
- einen (C₁-C₅)-Alkylrest, d.h. einen gerad- oder verzweigtkettigen gesättigten aliphatischen Kohlenwasserstoffrest, der 1 bis 5 Kohlenstoffatome enthält, wie Methyl, Ethyl, Propyl, Butyl, Pentyl,
Isopropyl, 1-Methylethyl, 1-Methylpropyl, 1-Methylbutyl, 2-Methylpropyl, 2-Methylbutyl oder 3-Methylbutyl, 1-Ethylpropyl, 2-Ethylpropyl;
- einen Fluoralkylrest, wie Monofluormethyl (-CH₂F) oder Difluormethyl (-CHF₂) oder Trifluormethyl (-CF₃) oder 1-Fluor-1-ethyl (-CHFCH₃) oder 1,1-Difluor-1-ethyl (-CF₂CH₃);
- einen Cyclopropyl- oder Cyclobutyl- oder Cyclopentylrest;
- eine substituierte oder unsubstituierte aromatische heterocyclische Gruppe mit 5 Kettengliedern, die 1, 2, 3 oder 4 Heteroatome enthält, die ausgewählt sind aus Stickstoff, Sauerstoff und Schwefel, jedoch ohne dass mehr als ein Sauerstoff- und/oder Schwefelatom in dem Heterocyclus A vorliegt,
- eine Alkoxy- (R₁O-) oder Alkylthiogruppe (R₁S-), in welcher der Rest R₁ darstellt:
· einen (C₁-C₅)-Alkylrest, wie vorstehend definiert,
· einen Monofluormethyl- oder Trifluormethylrest,
· einen Cyclopropyl- oder Cyclobutyl- oder Cyclopentylrest;
- eine Aminogruppe vom Typ II 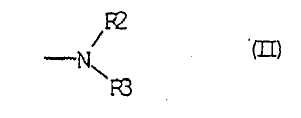 in der R₂ und R₃, identisch oder verschieden, Wasserstoff oder einen (C₁-C₅)-Alkylrest, wie vorstehend definiert, oder eine Cyclopropylgruppe oder eine Trifluormethylgruppe darstellen;
- eine gesättigte cyclische Aminogruppe vom Typ III 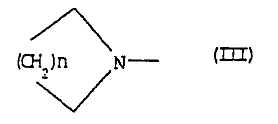 in der n die Werte von 1 oder 2 annehmen kann;
- eine Alkoxycarbonylgruppe, vorzugsweise eine Methoxycarbonylgruppe (CH₃OCO-) oder eine Ethoxycarbonylgruppe (CH₃CH₂OCO);
für die Herstellung eines Medikaments, das zur Behandlung der Arzneimittelabhängigkeit von Opioiden bestimmt ist.

2. Verwendung einer Verbindung der Formel (I) nach Anspruch 1, **dadurch gekennzeichnet, dass** A ein Wasserstoffatom darstellt, u ein Methyl darstellt, v und w ein Wasserstoffatom darstellen, x ein Fluor darstellt, y ein Chlor darstellt und z ein Fluor darstellt.
